Europäisches Patentamt

European Patent Office

Office européen des brevets

(19)

(11) Veröffentlichungsnummer: **0 146 055**
**B1**

(12)

# EUROPÄISCHE PATENTSCHRIFT

(45) Veröffentlichungstag der Patentschrift:
16.09.87

(51) Int. Cl.⁴: **A 61 B 6/04,** B 23 Q 1/16

(21) Anmeldenummer: **84114572.5**

(22) Anmeldetag: **30.11.84**

(54) **Röntgenuntersuchungstisch.**

(30) Priorität: **15.12.83 DE 3345434**

(43) Veröffentlichungstag der Anmeldung:
**26.06.85 Patentblatt 85/26**

(45) Bekanntmachung des Hinweises auf die Patenterteilung:
**16.09.87 Patentblatt 87/38**

(84) Benannte Vertragsstaaten:
**DE FR**

(56) Entgegenhaltungen:
**DE - B - 2 201 921**
**DE - C - 352 059**
**GB - A - 1 101 892**
**US - A - 2 007 563**
**US - A - 3 794 283**

(73) Patentinhaber: **Siemens Aktiengesellschaft Berlin und München, Wittelsbacherplatz 2, D-8000 München 2 (DE)**

(72) Erfinder: **Hahn, Alfred, Dipl.-Ing. (FH), Bunsenstrasse 64, D-8520 Erlangen (DE)**

## Beschreibung

Die Erfindung betrifft einen Röntgenuntersuchungstisch mit einem Tischgestell, an dem eine Patientenlagerungsplatte höhenverstellbar gelagert ist.

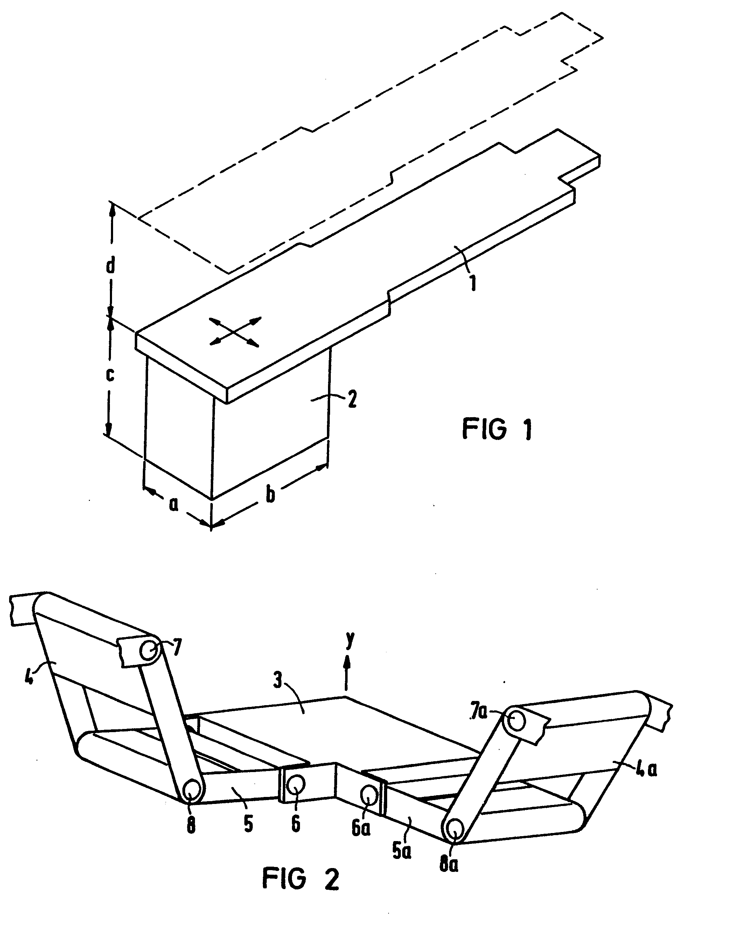

Röntgenuntersuchungstische dieser Art werden in der Angiographie zur Darstellung von Blutgefässen verwendet. Ein bekannter Untersuchungstisch für diesen Zweck ist in der Figur 1 dargestellt. Die Figur 1 zeigt eine Patientenlagerungsplatte 1, die in zwei zueinander senkrechten Richtungen in einer horizontalen Ebene und höhenverstellbar auf einem Tischgestell 2 gelagert ist. Der maximale, vertikale Hub aus der voll gezeichneten Ausgangsstellung in die gestrichelt gezeichnete Endstellung ist mit d bezeichnet. Die Abmessungen des Tischgestelles sind mit a, b und c angegeben.

Für die Angiographie wird gewünscht, dass die Masse a und b möglichst klein sind, damit der Patient für den Arzt von allen Seiten gut zugänglich ist. Ferner soll auch dass Mass c möglichst klein gehalten werden, d.h. die Patientenlagerungsplatte 1 soll möglichst weit abgesenkt werden können, damit eine leichte Umlagerung des Patienten von einem Bett auf die Patientenlagerungsplatte 1 möglich ist. Schliesslich soll das Mass d so gross sein, dass eine optimale Arbeitshöhe für den Arzt eingestellt werden kann.

Die geschilderten, optimalen Abmessungen sind bei den bekannten Röntgenuntersuchungstischen nur unvollkommen oder mit hohen Kosten, z.B. bei einer Deckenaufhängung, erreicht.

Der Erfindung liegt die Aufgabe zugrunde, einen Röntgenuntersuchungstisch der eingangs genannten Art so auszubilden, dass mit einfachen Konstruktionselementen eine kleine Grundfläche des Tischgestelles, eine niedrige, minimale Tischhöhe und eine optimale Arbeitshöhe erreichbar sind.

Diese Aufgabe ist erfindungsgemäss dadurch gelöst, dass die Patientenlagerungsplatte an einem Hubkörper befestigt ist, mit dem zwei Doppelgelenkträger um zwei senkrecht zueinander liegende horizontale Achsen gelenkig verbunden sind, von denen jeder zwei Gelenkarme aufweist, die um zwei parallele, horizontale Achsen schwenkbar sind, die parallel zur Gelenkachse am Hubkörper liegen, dass die Schwenkachsen der beiden Doppelgelenkträger senkrecht zueinander liegen und dass am Hubkörper ein Höhenverstellmechanismus angreift. Die Elemente, aus denen die beiden Doppelgelenkträger bestehen, können von ihren Abmessungen her bei ausreichendem Hub des Hubkörpers klein gehalten werden und nehmen daher wenig Platz im Tischgestell ein. Der Höhenverstellmechanismus kann in einfacher Weise unterhalb des Hubkörpers angeordnet werden, wo Platz zur Verfügung steht.

Eine zweckmässige Ausgestaltung des Röntgenuntersuchungstisches nach der Erfindung besteht darin, dass der Höhenverstellmechanismus von einer motorisch ausfahrbaren, unterhalb des Hubkörpers liegenden Säule gebildet ist. Diese Säule kann aus mehreren ineinandersteckenden Hubspindeln bestehen, die mit ineinandergreifenden Innen- und Aussengewinden versehen sind. Die innerste Hubspindel kann dabei motorisch drehbar sein. Dadurch ist bei niedriger minimaler Bauhöhe ein optimaler Hub möglich.

Die Erfindung ist nachfolgend anhand der Zeichnungen näher erläutert. Es zeigen:

Fig. 2 die wesentlichen Teile eines Röntgenuntersuchungstisches nach der Erfindung,

Fig. 3 einen Röntgenuntersuchungstisch nach der Erfindung in einer Seitenansicht,

Fig. 4 eine Draufsicht auf das Tischgestell des Röntgenuntersuchungstisches gemäss Figur 3,

Fig. 5 den Höhenverstellmechanismus für den Röntgenuntersuchungstisch gemäss den Figuren 3 und 4.

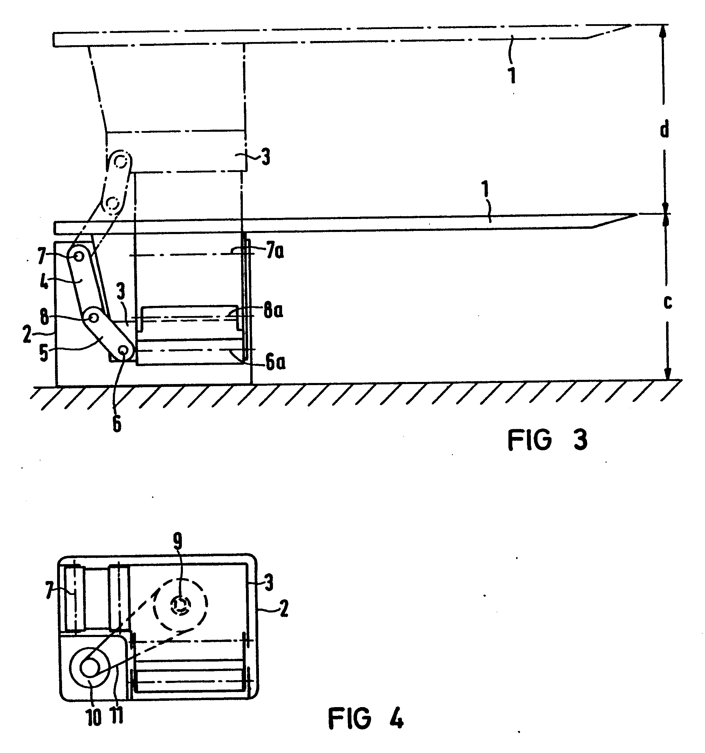

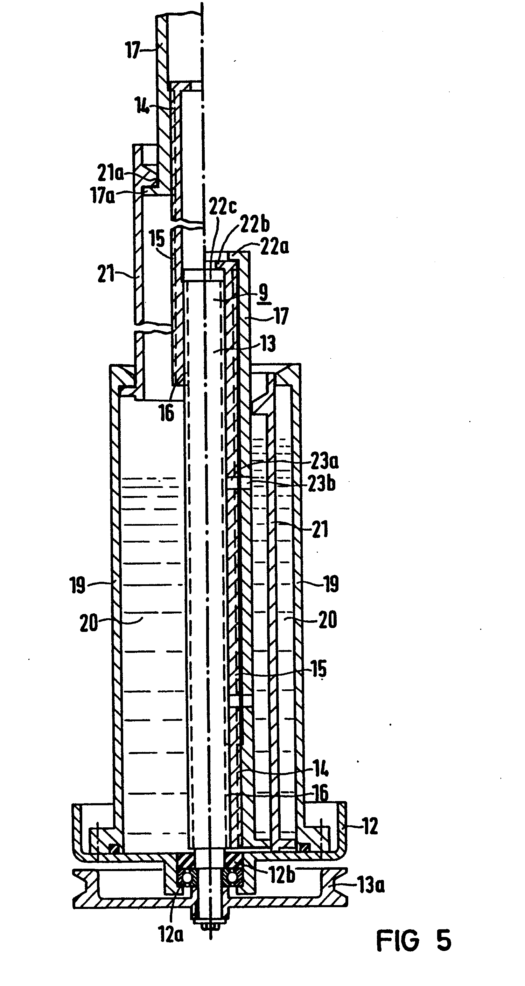

In der Figur 2 ist schematisch ein Hubkörper 3 dargestellt, mit dem die Patientenlagerungsplatte 1 verbunden ist, und an dem zwei Doppelgelenkträger 4, 5; 4a, 5a um zwei senkrecht zueinanderliegende horizontale Achsen 6, 6a schwenkbar angelenkt sind. Jeder der Doppelgelenkträger weist zwei Gelenkarme 4, 5 bzw. 4a, 5a auf, die um zwei parallele, horizontale Achsen 7, 8; 7a, 8a schwenkbar sind, die parallel zur jeweiligen Gelenkachse 6, 6a am Hubkörper 3 liegen. Die Schwenkachsen 7, 8; 7a, 8a der beiden Doppelgelenkträger 4, 5; 4a, 5a liegen senkrecht zueinander. Die Achsen 7, 7a sind dabei gerätefest. Der Gelenkmechanismus gemäss der Figur 2 ermöglicht lediglich eine Verstellung des Hubkörpers 3 in vertikaler Richtung, die in der Figur 2 mit y bezeichnet ist. Diese Verstellung kann durch einen in Verbindung mit den Figuren 4 und 5 näher erläuterten Höhenverstellmechanismus erfolgen, der unterhalb des Hubkörpers 3 angeordnet ist.

Die Figur 3 zeigt die Anordnung des Gelenkmechanismus gemäss Figur 2 im Tischgestell 2, und zwar in einer voll gezeichneten niedrigsten Stellung der Patientenlagerungsplatte 1 und in einer stichpunktiert gezeichneten höchsten Stellung der Patientenlagerungsplatte 1. Aus der Figur 3 geht hervor, dass bei geringem Raumbedarf für den Gelenkmechanismus ein ausreichend grosser Hub d zur Verfügung steht. Dass Mass c kann dabei sehr klein gehalten werden.

Aus der Figur 4 geht hervor, dass unterhalb des Hubkörpers 3 ein Höhenverstellmechanismus angeordnet ist, der aus einer ausfahrbaren Säule 9 besteht, die von einem Elektromotor 10 über einen Keilriemen 11 angetrieben wird. Der Elektromotor 10 liegt dabei im Raum zwischen den beiden Doppelgelenkträgern 4, 5; 4a, 5a.

Die Figur 5 zeigt, dass die Säule 9 eine erste Hubspindel 13 aufweist, die in einem Fusskörper 12 mit Hilfe eines Lagers 12a drehbar gelagert ist. Eine Dichtung 12b dichtet dabei den Raum oberhalb des Fusskörpers 12 öldicht ab. Die Hubspindel 13 wird von einem Rad 13a über den Keilriemen 11 gedreht. Sie steht mit ihrem Aussengewinde mit dem Innengewinde 16 einer zweiten Hubspindel 15 in Eingriff. Die Hubspindel 15 steht mit ihrem Aussengewinde 14 mit dem Innengewinde eines Rohres 17 in Eingriff, das am Hubkörper 3 befestigt ist.

Die Schmierung wird über in einem Ölgefäss 19 enthaltenes Schmieröl 20 gewährleistet. Ein Spritzölfänger 21 führt das bei vollem Hub von der Hubspindel 15 abspritzende Öl in das Ölgefäss 19 zurück.

In der rechten Hälfte der Figur 5 sind die Komponenten 13, 15, 17, 21 in ihrer Ausgangsstellung gezeigt, in der die Patientenlagerungsplatte 1 die in der Figur 3 voll gezeichnete tiefste Stellung einnimmt. Soll die Patientenlagerungsplatte 1 nach oben verstellt werden, so treibt der Motor 10 die Hubspindel 13 an, wobei sich die Hubspindel 15 und das Rohr 17 nach oben bewegen. In der linken Hälfte der Figur 5 sind die obersten Stellungen der Komponenten 15 und 17 gezeigt. Bei der Bewegung des Rohres 17 nach oben wird dabei der hülsenförmige Spritzölfänger 21 durch einen Anschlag 17a am Rohr 17 und einen Anschlag 21a am Spritzölfänger 21 mitgenommen. Anschläge 22a, 22b und 22c dienen zur Wegbegrenzung der Komponenten 13, 15, 17. Bohrungen 23a, 23b sorgen dafür, dass die Gewinderäume immer mit Öl gefüllt sind.

**Patentansprüche**

1. Röntgenuntersuchungstisch mit einem Tischgestell (2), an dem eine Patientenlagerungsplatte (1) zwischen einer oberen und einer unteren Endlage mittels eines Höhenverstellmechanismus höhenverstellbar gelagert ist, wobei die Patientenlagerungsplatte (1) an einem Hubkörper (3) befestigt ist, dadurch gekennzeichnet, dass mit dem Hubkörper (3) zwei Doppelgelenkträger (4, 5; 4a, 5a) um zwei senkrecht zueinanderliegende, horizontale erste Achsen (6, 6a) gelenkig verbunden sind, von denen jeder zwei Gelenkarme (4, 5; 4a, 5a) aufweist, die jeweils um eine zweite und eine dritte parallele, horizontale Achse (8, 7; 8a, 7a) schwenkbar sind, die parallel zur jeweils entsprechenden ersten Achse (6, 6a) und jeweils senkrecht zueinander liegen, wobei die dritten Achsen (7, 7a) an dem Tischgestell (2) befestigt sind, dass sich die ersten und zweiten Achsen (6, 8; 6a, 8a) in der oberen Endlage der Patientenlagerungsplatte oberhalb und in deren unterer Endlage unterhalb der dritten Achsen (7, 7a) befinden und dass der Höhenverstellmechanismus am Hubkörper (3) angreift.

2. Röntgenuntersuchungstisch nach Anspruch 1, dadurch gekennzeichnet, dass der Höhenverstellmechanismus von einer motorisch ausfahrbaren, unterhalb des Hubkörpers (3) liegenden Säule (9) gebildet ist.

3. Röntgenuntersuchungstisch nach Anspruch 2, dadurch gekennzeichnet, dass die Säule (9) aus mehreren ineinandersteckenden Hubspindeln (13, 15, 17) besteht, die mit ineinander eingreifenden Innen- und Aussengewinden versehen sind, und dass die innerste Hubspindel (13) motorisch drehbar ist.

**Claims**

1. An x-ray examination table comprising a table mount (2) on which a patient support plate (1) is mounted so as to be height-adjustable between an upper and lower end position by means of a height adjusting mechanism, wherein the patient support plate (1) is secured to a lifting component (3), characterised in that the lifting component (3) is articulated to two double-link carriers (4, 5; 4a, 5a) in such manner that these can be pivoted about two horizontal first axes (6, 6a) which are arranged at right angles to one another, where each of the double-link carriers is provided with two articulated arms (4, 5; 4a, 5a), each of which can be pivoted about a second and third, parallel, horizontal axis (8, 7; 8a, 7a) which extends in parallel to the respective corresponding first axis (6, 6a) and at right angles to one another, where the third axes (7, 7a) are fixed to the table mount (2), that the first and second axes (6, 8; 6a, 8a) are located above the third axes (7, 7a) when the patient support plate is in the extreme upper position and below the third axes (7, 7a) when the patient support plate is in its extreme lower position, and that the height adjusting mechanism acts upon the lifting component (3).

2. An x-ray examination table as claimed in Claim 1, characterised in that the height adjusting mechanism is constituted by a column (9) which can be extended under the influence of a motor, and which is located below the lifting component (3).

3. An x-ray examination table as claimed in Claim 2, characterised in that the column (9) consists of a plurality of nesting lifting spindles (13, 15, 17) which are provided with inter-engaging inner and outer threads, and that the innermost lifting spindle (13) can be rotated by a motor.

**Revendications**

1. Table d'examen radiologique comportant un bâti de table (2), sur lequel un plateau (1) formant couchette pour patient est monté de façon à pouvoir être réglé en hauteur entre une position limite supérieure et une position limite inférieure, à l'aide d'un mécanisme de réglage en hauteur, le plateau (1) formant couchette pour patient étant fixé à un corps relevable (3), caractérisée par le fait qu'au corps relevable (3) se trouvent reliés de façon articulée, de manière à pouvoir pivoter autour de deux premiers axes horizontaux (6, 6a) perpendiculaires entre eux, des supports articulés doubles (4, 5; 4a, 5a), dont chacun comporte deux bras articulés (4, 5; 4a, 5a), qui peuvent pivoter respectivement autour de seconds et troisièmes axes horizontaux parallèles (8, 7; 8a, 7a) qui sont parallèles au premier axe respectivement correspondant (6, 6a) et sont perpendiculaires entre eux, les troisièmes axes (7, 7a) étant fixés sur le bâti (2) de la table, que les premiers et seconds axes (6, 8; 6a, 8a) sont situés au-dessus des troisièmes axes (7, 7a) lorsque le plateau formant couchette pour patient est dans sa position limite supérieure, et au-dessous de ces axes lorsque le plateau formant couchette pour patient est dans sa position limite inférieure et que le mécanisme de réglage en hauteur actionne le corps relevable (3).

2. Table d'examen radiologique suivant le revendication 1, caractérisé par le fait que le mécanisme de réglage en hauteur est formé par une colonne (9) pouvant être déployée au moyen d'un moteur et située au-dessous du corps relevable (3).

3. Table d'examen radiologique suivant la revendication 2, caractérisé par le fait que la colonne (9) est constituée par plusieurs broches de levage (13, 15, 17) engagées les unes dans les autres et qui sont munies de taraudages et de filetages extérieurs de levage la plus intérieure (13) peut être entraînée en rotation par le moteur.

FIG 1

FIG 2

**FIG 3**

**FIG 4**

FIG 5